# EUROPEAN PATENT APPLICATION

(11) **EP 4 652 915 A1**
(43) Date of publication of application: **26.11.2025**
(21) Application number: 25175841.3
(22) Date of filing: 12.05.2025
(51) Int. Cl.: A61B 1/00

(54) **IMAGING DEVICE AND SYSTEM**

(30) Priority: 21.05.2024 US 202418670020
(71) Applicant: KARL STORZ SE & Co. KG, 78532 Tuttlingen (DE)
(72) Inventor: Jonathan, Bormet, Goleta, CA 93117 (US)

(57) **Abstract**

An imaging device for obtaining an image of an interior portion of a patient includes a body having a proximal end and a distal end configured to be inserted into the patient. The imaging device also includes an optical system including an image sensor and an optical lens configured to capture images along an optical axis defined by a longitudinal axis of the body. Additionally, the imaging device includes a reflective element surrounding at least a portion of the optical axis. Moreover, the reflective element is cylindrically or conically shaped. Additionally, the optical lens is interposed between the image sensor and the reflective element and configured to transmit light from the reflective element onto the image sensor.

## Description

### TECHNICAL FIELD

The present disclosure relates generally to the field of an imaging device and system and more specifically to a stereoscopic imaging device and system and a method of displaying an image.

### BACKGROUND

Imaging devices, such as endoscopes and exoscopes, provide doctors and other medical personnel with images of the inside of a patient's body to examine or treat organs or structures. The imaging device may be rotated during use, in which case the displayed image is also rotated which may not be desirable by the surgeon. Accordingly, imaging systems may include an auto-level function which maintains the displayed image in a desired orientation. For instance, the auto-level function may be configured to maintain the image in a horizontal orientation.

Imaging devices may be configured to generate three dimensional views. In such an aspect, the imaging device is stereoscopic and includes a left camera and a right camera that are laterally offset from each other in a fixed relationship so as to capture a left-side image and a right-side image. The left-side image and right-side image captured by the cameras are processed by a controller and displayed as three-dimensional (3D) stereo images by overlaying one image onto the other. As the endoscope is physically rotated, the corresponding left and right images may not only be rotated, but may also be moved. Thus, performing auto-leveling may not necessarily be done by simply rotating the respective left-side and right-side images. For instance, assuming that the endoscope is rotated about the left camera, the left-side image may be simply rotated; however, as the right camera is rotated about the left camera, the right-side image is moved. Thus, if both the left-side image and the right-side image are simply rotated and overlaid onto each other, the resulting three-dimensional image will be distorted. Likewise, should the endoscope be rotated about an axis centered between the left camera and right camera, both of left-side image and the right-side image are moved in which case simply rotating each image will result in a distorted three-dimensional image.

Further, conventional imaging devices require two channels, or two cameras, defining a left eye view and a right eye view. Such a configuration increases the profile of the scope, and thus, the diameter of the incision must be large enough to accommodate the scope.

Catadioptric imaging systems and methods are known from US7,420,750B2 and CN104434001B. An optical imaging device with a distal mirror is disclosed in US11,042,014B2 and US2021/003836A1. An image distortion transformation method for transforming an original image by an imager is disclosed in US10,748,243B2. A scanning endoscope with a mirror is shown in US2010/0125170A1.
Further prior art is disclosed in: Paulson, Bjorn et al., Miniaturized omnidirectional flexible side-view endoscope for rapid monitoring of thin tubular biostructures, Biomedical Optics Express 2264, Vol. 10, No. 5, May 1, 2019, 11 pgs.;
Kuthirummal, Sujit et al., Multiview Radial Catadioptric Imaging for Scene Capture;
Gurrieri, Luis E. et al, Acquisition of omnidirectional stereoscopic images and videos of dynamic scenes: a review. SPIE, Journal of Electronic Imaging, 22(3), 030902 (Jul-Sep 2013); and Jang, Gijeong et al., Single Camera Catadioptric Stereo System, <https://www.researchgate.net/publication/228359132,> January 2005.

Accordingly, it is desirable to provide a stereoscopic imaging system configured to perform auto-leveling during rotation and is configured as a single channel device wherein a single optical system and image sensor is used to generate a three-dimensional image, so as to reduce the size of an incision.

### SUMMARY

In one configuration, an imaging device for obtaining an image of an interior portion of a patient includes a body having a proximal end and a distal end configured to be inserted into the patient and an optical system including an image sensor and an optical lens configured to capture images along an optical axis defined by a longitudinal axis of the body. The imaging device also includes a reflective element surrounding at least a portion of the optical axis and the reflective element is cylindrically or conically shaped. Additionally, the optical lens is interposed between the image sensor and the reflective element and configured to transmit light from the reflective element onto the image sensor.

The imaging device may be an endoscope for viewing the interior of a patient. The endoscope can be a rigid or a flexible instrument and may be suitable for different imaging modalities such as white light or fluorescence imaging. The imaging device can also be a microscope or exoscope for viewing a patient from the outside. The body may include a scope portion and a handle portion. The scope portion may include the optical lens. The optical lens may comprise one or several lenses that form an optical system in the device, such as an objective lens assembly or a relay lens group. The body of the device may include a longitudinal shaft. The image sensor can be disposed at a distal or a proximal portion of the device. It may be part of the device or located in a separate camera head unit. The reflective element may be a mirror that is located inside or at an end of the device such that it at least partially surrounds the optical axis. It may be a conical element or mirror that has a wider and a narrower end, where the narrower end has an opening that is open towards the proximal end and/or the image sensor, and where the wider end has an opening that is open towards the distal end of the device, or where light enters. Light entering the reflective element from different angles is reflected by the inner reflective surface and at least partially reflected towards the image sensor. Therefore, light from an object or a scene in front of the device can be captured by the image sensor at the same time, showing different perspectives of the object or scene. A central image and a plurality of distorted images surrounding the central image and forming a composite image will be created on the image sensor.

The imaging device may also include one or more of the following optional features. As is known in the art, the imaging device may be of a straight looking type or the main viewing direction may be at an angle of, for example, 15, 30, 45 or 60 degrees. For example, the reflective element may be attached to the distal end of the body. This allows for a larger reflective element / mirror that is not confined to the inner diameter of the imaging device. In one aspect, the reflective element may be spaced apart from the distal end. Therefore, the reflective element does not have to be located directly at the distal end of the imaging device. In some examples, the reflective element is configured to be moveable between a collapsed position and an extended position. For example, this allows for the reflective element to be located inside the imaging device in a collapsed position and outside the device in an extended position, where the reflective element is unfolded. This allows for the reflective element to be located inside the device during insertion into the patient and outside the device after insertion in the case of an endoscope. The reflective element can therefore be larger than the imaging device diameter without having to create a larger incision in the patient. The reflective element could be moved into an extended position like an umbrella. Various mechanisms for moving and/or deploying the reflective element, such as a push-pull-rod, are conceivable. In some implementations, the reflective element is at least partially disposed within the distal end. Additionally, the reflective element may be a cylindrical mirror. In some examples, the reflective element is a conical mirror. Both shapes allow for the advantageous imaging of an object or scene in front of the imaging device from different perspectives.

In another configuration, an imaging system for obtaining images of an interior portion of a patient includes an imaging device configured to operate at an angle of operation. That is, the imaging device may be rotated during operation and therefore, be at different angles of operation. The imaging device includes a body having a proximal end and a distal end, that may be configured to be inserted into the interior portion of the patient. The proximal end will then be located outside of the patient during the medical application. The imaging device also includes an optical system including an image sensor and an optical lens configured to capture images along an optical axis defined by a longitudinal axis of the body. Additionally, the imaging device includes a reflective element configured to at least partially surround the optical axis, the reflective element being one of a cylindrical or conical shape so as to generate a plurality of images from a plurality of perspectives, the plurality of images captured by the image sensor. The imaging system also includes an image processor configured to process the angle of operation of the imaging device and select one of the plurality of images corresponding to the angle of operation, the selected image and a central image is processed to generate a three-dimensional image corresponding to the angle of operation. The imaging system further includes a display configured to display the three-dimensional image. The imaging system may include a camera control unit (CCU) that includes the image processor. The image processor may also include several components that can be partially located in the camera control unit and/or the imaging device itself. The system can also be a stand-alone system where all components are part of the imaging device. The display may be an external display connected to the imaging device and / or the image processor. It can also be located directly on the imaging device. The display is configured so that it can display three dimensional images either for direct viewing or for viewing with additional glasses.

The imaging system may also include one or more of the following optional features. For example, the imaging system may also include an orientation detection unit such as an inertial sensor or a gyroscope configured to sense the angle of operation of the imaging device. The orientation detection unit can be or include any component suitable for detecting a rotational or other position of the imaging device. Additionally, the image processor may be configured to select the pair of images according to a desired angle of operation of the imaging device. The imaging system described here simultaneously images several images from different perspectives of an object located in front of the imaging device. The image processor may select two of those images to create the three-dimensional image such that the image remains at a fixed orientation even if the angle of operation of the imaging device changes. That is, the images are chosen depending on the detected orientation of the instrument, such that the three-dimensional image has a desired orientation or perspective. In some implementations, the imaging system also includes an input for setting a desired perspective, which may include a desired orientation of the three-dimensional image, wherein the image processor is configured to process the desired perspective and the angle of operation of the imaging device to select the pair of images. The input may include buttons, a touchscreen or the like. In some examples, the image processor is configured to process a difference in images between at least a pair of successive image frames to determine the pair of images corresponding to the angle of operation. Additionally, the image processor may be configured to automatically perform stereoscopic image rotation by choosing a desired horizon and then determining the pair of images that correspond to the desired horizon. The horizon may be the actual horizon, i.e. the images may be chosen such that the three-dimensional image is upright with regards to the direction of gravitation. Alternatively, a user may set the system to maintain a horizon that differs from the actual horizon. Maintaining an image horizon during rotation of the imaging device is also referred to as auto-leveling. The image rotation may be performed in real-time, so that the image always appears to the user with the desired orientation. In some implementations, the image processor is configured to extract dimensions of an object using triangulation. As is known in the art, imaging systems and specifically stereo imaging systems can be used to triangulate the position and depth of object points and be used to calculate distances and dimensions in an image. Additionally, the image processor is further configured to detect an illumination in a plurality of images and select the pair of images that do not have a specular reflection and correspond to the angle of operation. In other words, if a reflection of light from an illumination source is detected in one or more of the images that, for example, disturbs the image, the pair of images can be chosen such that the reflection is no longer visible in the image.

Moreover, in some implementations, a method of displaying an image from an imaging device having a body includes obtaining a plurality of images from a plurality of perspectives from a reflective element and the reflective element being one of a cylindrical or conical shape. The method also includes determining an orientation of the imaging device and processing the orientation of the imaging device. Additionally, the method includes selecting a left image and a right image from the plurality of images, wherein one of the left image and the right image corresponds to a central image and the other of the left image and the right image is one of the plurality of images and displaying the left image and the right image on a display.

As described above, the reflective element can be a mirror. The features of the imaging device and system and the function of the device and system mentioned above also apply to the imaging device used in the method. The orientation can be determined using an orientation detection unit and an image processor as described above.

The method may include one or more of the following features. For example, the step of processing the left image and the right image may include superimposing the left image onto the right image to create a stereoscopic image. Additionally, in some examples, both the left image and the right image may include a first image and a second image, and the method may include the step of processing a difference in the first image and the second image to select a subsequent left view image and right view image. In some implementations, the step of analyzing the left image and the right image may use triangulation to extract dimensions of an object. In some examples, the step of processing the plurality of images includes to select the left image and right image, wherein the selected left image and right image have an illumination that is not reflected directly into an optical lens.

In another aspect, an imaging system for obtaining images of an interior portion of a patient includes an imaging device. The imaging device includes a body having a proximal end and a distal end configured to be inserted into the interior portion of the patient. The imaging system includes an optical system having an image sensor and an optical lens configured to capture a real image along an optical axis defined by a longitudinal axis of the body. The imaging system includes a reflective element configured to surround the optical axis. The reflective element being one of a cylindrical or conical shape so as to generate a plurality of virtual images from a plurality of perspectives, the plurality of virtual images and the real image captured by the image sensor. An image processor is configured to process the plurality of virtual images and the real image to generate a plurality of perspective view of the interior portion. The imaging system further includes a display configured to display the plurality of perspectives.

### BRIEF DESCRIPTION OF THE DRAWINGS

The drawings described herein are for illustrative purposes only of selected configurations and are not intended to limit the scope of the present disclosure.
FIG. 1 is a perspective view of a video imaging system according to the present disclosure viewing objects within a scene;
FIG. 2 is a schematic view of the video imaging system shown in FIG. 1;
FIG. 3A is depiction of the imaging device shown in FIG. 2 showing the operation of the reflective element;
FIG. 3B is cross-sectional view of FIG. 3A taken along the longitudinal axis of the imaging device;
FIG. 3C is a diagram showing the structure of a composite image generated by the imaging device shown in FIGS. 3A and 3B;
FIG. 4 is an illustrative view of a composite image generated by the imaging device shown in FIGS. 3A and 3B;
FIG. 5A cross-sectional view of the imaging device shown in FIG. 2 taken along the longitudinal axis of the imaging device;
FIG. 5B is a cross-section view of an imaging device configured to provide a view that is angled with respect to the longitudinal axis of the imaging device;
FIG. 6A is a cross-sectional view of the imaging device wherein the reflective element is disposed within the scope portion;
FIG. 6B a cross-section view of an imaging device wherein the reflective element is disposed within the scope portion and is configured to provide a view that is angled with respect to the longitudinal axis of the imaging device;
FIG. 6C cross-section view of an imaging device wherein the reflective element is disposed within the scope portion and the length of the reflective element is shorter relative to the reflective element shown in FIG. 6A;
FIG. 7A is a side view of an imaging device according to one or more aspects disclosed herein;
FIG. 7B is a plan view of the reflective element;
FIG. 8A is a view of a composite image showing the radial line;
FIG. 8B is a view of FIG. 8A showing the position of the left and right eye;
FIG. 8C is a view of FIG. 8B showing the position of the left and right eye rotated counter-clockwise relative to FIG. 8B;
FIG. 8D is a view of FIG. 8C showing the position of the left and right eye rotated counter-clockwise relative to FIG. 8C;
FIG. 9A is a view of a composite image showing a stereoscopic base line defined by a right eye being centered and a left eye at a three o'clock position;
FIG. 9B is a view of a composite image showing a stereoscopic base line defined by a left eye being centered and a right eye at a nine o'clock position;
FIG. 9C is a view of a composite image showing a stereoscopic base line defined by a right eye being at a nine o'clock position and a left eye at a three o'clock position; and
FIG. 10 is a flow chart showing the steps of a method displaying an image generated by an imaging device having a reflective element.

Corresponding reference numerals indicate corresponding parts throughout the drawings.

### DETAILED DESCRIPTION

Example configurations will now be described more fully with reference to the accompanying drawings.

An imaging system for generating a three-dimensional image of an interior portion of a patient includes an imaging device configured to operate at an angle of operation and an optical system including an image sensor, an optical lens, and a reflective element configured to generate a plurality of images from a plurality of perspectives, the plurality of images captured by the image sensor. An image processor processes the angle of operation of the imaging device and selects a pair of images corresponding to the angle of operation; and a display is configured to display the selected pair of images corresponding to the angle of operation as a three-dimensional image so as to auto-level the three-dimensional image.

The imaging system as described herein uses the reflective element to simultaneously capture image data, both real and virtual, that correspond to a plurality of perspectives in a single composite image including a distorted image, formed of a plurality of virtual images taken at different perspective, bounding a real image. The composite image is processed wherein a virtual image may be synthesized with another virtual image or the real image to generate a realistic view of the scene that is taken from a perspective other than the perspective of the imaging device. Further, since the virtual images of the composite image are formed around the real image, stereoscopic views corresponding to arbitrary horizon rotation angles can be generated. Further, the imaging system may include an auto leveling function to support three-dimensional horizon stabilization by compensating for physical rotation of the scope with synthetically rotated views, wherein a region of interest from the distorted image corresponding to the corrected angle may be used to synthesize views having a desired perspective.

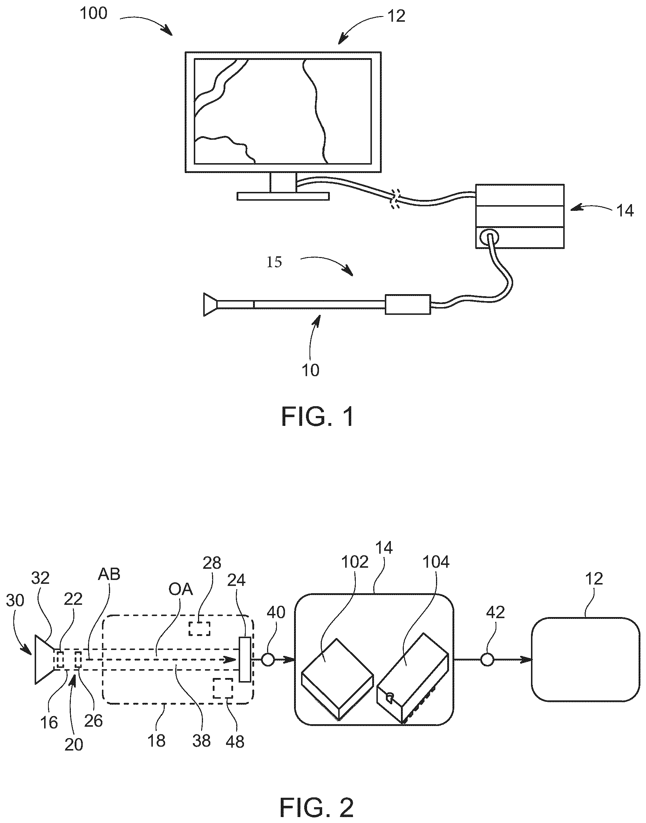

With reference now to FIG. 1, a schematic depiction of a video imaging system 100 is provided. For illustrative purposes, the video imaging system 100 is discussed in the context of a surgical procedure, but it should be appreciated that the video imaging system 100 may be used for other applications. The video imaging system 100 includes an imaging device 10 and a display 12. For illustrative purposes, the imaging device 10 is shown as a video endoscope and is coupled to a camera control unit (CCU) 14 which functions as an image processor in addition to providing camera control functions such as zooming, changing the view, adjusting light, adjusting exposure and the like. In one aspect, the imaging device 10 is detachably coupled to the CCU 14. The imaging device 10 is configured to capture images of an interior of a body of a patient and the CCU 14 is configured to perform image processing to generate a still or video image for production on the display 12.

With reference now to FIG. 2, a depiction of the imaging device 10 is provided. The imaging device 10 includes a scope portion 16 and a handle portion 18. The scope portion 16 is configured to be inserted into the patient and the handle portion 18 is configured to be held by a user. The imaging device 10 further includes an optical system 20 having components which are housed in the scope portion 16 and the handle portion 18. The optical system 20 includes an objective lens assembly 22 and an image sensor 24. The objective lens assembly 22 is configured to collect and focus image light from a scene 200 under observation onto the image sensor 24. The optical system 20 may further include a relay lens group 26 that is configured to relay image light collected by the objective lens assembly 22 to the image sensor 24. In one aspect, the image sensor 24 may be a complementary metal oxide semiconductor (CMOS) or a Charged Coupled Device (CCD). It should be appreciated that any pixelated image sensor 24 currently known or later developed may be modified and adopted for use herein. The scope portion 16 may have a generally cylindrical shape having a longitudinal axis "AB". As shown in FIG. 2, light collected by the objective assembly is transmitted to the image sensor 24 along an optical axis "OA" that is coaxial with the longitudinal axis "AB" of the scope portion 16. The imaging device 10 may further include a camera head unit 28 which may be fixedly attached or integrally formed with the handle portion 18. The camera head unit 28 is configured to control various camera functions such as moving the objective lens assembly 22 to zoom in or out of the surgical scene 200.

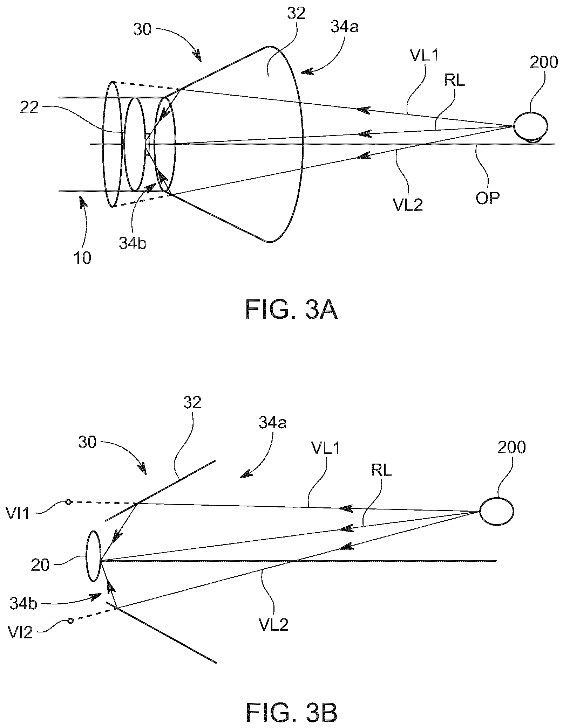

With reference again to FIGS. 1 and 2, and now to FIG. 3A, the imaging device 10 further includes a reflective element 30 disposed adjacent a distal end of the scope portion 16. The reflective element 30 partially surrounds a portion of the body of the scope portion 16. The reflective element 30 includes a peripheral wall 32 that may be a cylindrical or conical shape so as collect a plurality of images from a plurality of perspectives. As shown in FIG. 3A, the reflective element 30 includes an opening 34a, 34b at both ends of the peripheral wall 32 so as to clear an optical path "OP" of the scene 200 to the optical system 20. In this case, the scene 200 is offset from the optical path "OP", and as such, the light rays from the scene 200, referenced herein as "real light rays" and labeled as "RL", are angled relative to the optical path "OP". The real light rays "RL" from the scene 200 are gathered by the image sensor 24 and are processed by the CCU 14 to provide a real view of the scene 200. The reflective element 30 gathers a plurality of virtual images "VI" which are generated by virtual light rays "VL" of the scene 200 reflected off of the inner surface of the reflective element 30 onto the optical system 20, as indicated by the lines labeled "VL1" and "VL2". The virtual light rays "VL1", "VL2", VL_{N} are images of the scene 200 taken at different perspectives. It should be noted that as the reflective element 30 surrounds the optical path "OP", there are a plurality of virtual light rays VL_{N} reflecting along the length of the reflective element 30, thus generating a plurality of virtual images, but only two virtual light rays are shown, as identifying each virtual light ray would obscure the drawing.

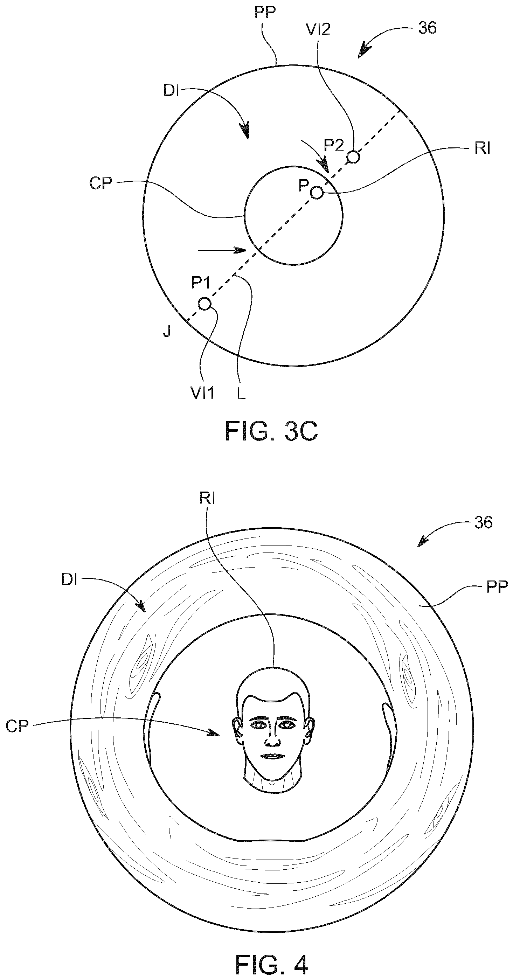

With reference now to FIGS. 3B and 3C, a description of the image generated by the imaging device 10 is provided. FIG. 3B provides a cross-sectional view of FIG. 3A which shows that the real image "RI" and the virtual images "VI" are generated by the real light rays "RL" and the virtual light rays "VL1", "VL2", "VL_{N}" gathered by the imaging device 10. FIG. 3B illustrates how the virtual light rays "VL1", "VL2" are received by the imaging device 10 at different angles as the scene 200 is offset from the optical path "OP". Even though virtual light rays "VL1" and "VL2" are at different angles, the virtual images VI generated by virtual light rays "VL1", "VL2" are symmetric with respect to the optical path "OP". FIG. 3B also illustrates how each virtual image generated by the imaging device 10 has a counterpart that is symmetrical about the optical path "OP" even if the angle of reflection of the virtual rays of light "VL1", "VL2" are not reflected at the same angle.

FIG. 3C illustrates the image generated by the imaging device 10. The image is a composite image 36 having a center portion "CP" in which the real image "RI" is displayed and a peripheral portion "PP" that is concentric to the center portion "CP" in which a plurality of virtual images "VI" are displayed. It should be appreciated that the virtual images "VI" are of the scene 200 taken at different perspectives. Accordingly, the virtual images "VI" include content that may not be present in the real image "RI". Virtual images "VI1", "VI2" and real image "RI" are fixed along a radial line "L". Although the virtual images "VI1" and "VI2" are symmetric to each other optically, they are spaced apart from the real image "RI" at different distances. Thus, the resulting image displayed in the peripheral portion PP is a distorted image "DI" of the scene 200 formed of a plurality of virtual images "VI", as shown in FIG. 4. Further, as the virtual images "VI" are of the scene taken from different perspectives, there is content of the scene that is not available from a head-on view presented by the real image "RI". However, the real image "RI" and the virtual images "VI" include image content that correspond to each other and correspond to the scene.

In operation, the real image "RI" and the virtual images "VI_{N}" (collectively referenced herein as the "image data 38"), are collected by the image sensor 24 which converts the image data 38 into objective lens data 40 which is then transmitted to the CCU 14. The objective lens data 40 is processed by the CCU 14 to generate the composite image 36 illustratively shown in FIG. 4. The CCU 14 includes a data processing hardware 102 which executes instructions or programs stored on a memory hardware 104 for processing the objective lens data 40 to generate a two-dimensional or three-dimensional image frame 42, as the case may be. In another aspect, as discussed in greater detail below, the CCU 14 may be further configured to select at least one of the virtual images from the distorted portion of the objective lens data 40 that corresponds to an angle of orientation of the imaging device 10. It should be noted that the CCU 14 may be an element of the imaging device 10 or a separate unit as shown in FIG. 2. For example, the imaging device 10 may include the objective lens assembly 22, the relay lens group 26, the image sensor 24, the data processing hardware 102, and the memory hardware 104. Alternatively, the camera head unit 28 may be detachably connected to the imaging device 10 or integrally formed with the imaging device 10.

With reference again to FIG. 1, the video imaging device 10 is configured to generate a plurality of image frames 42 which may be compiled together to form a video image for production on the display 12. As shown in FIGS. 3A and 3B, the image data 38 is in the form of light rays, e.g. virtual light rays "VL_{N}" and real light rays "RL_{N}" which includes a plurality of virtual images "VI" and at least one real image "RI", wherein the virtual images "VI" are generated by virtual light rays "VL_{N}" reflected by the reflective element 30 onto the image sensor 24 and the real image is an unimpeded or non-reflected ray of light. The real and virtual rays of light optically generate the composite image 36 shown in FIG. 4 and the composite image 36 is processed by the data processing hardware 102 disposed in the CCU 14. The CCU 14 includes the memory hardware 104 which stores instructions executable by the data processing hardware 102 which not only generates an image frame 42 but also performs image processing such as frame optimization, improved dynamic range, noise reduction, bandwidth filtering, edge detection, and the like so as to generate an image frame 42 as discussed in greater detail below. The image frames 42 are transmitted to the display 12 in the form of the video image.

Referring again to FIG. 2, the video imaging system 100 further includes an orientation detection unit 44 configured to sense an angle of operation of the imaging device 10. The orientation detection unit 44 may be an inertial sensor or gyroscope. The CCU 14 is further configured to process the angle of operation of the imaging device 10 detected by the orientation detection unit 44 and select a pair of images, virtual and/or real, corresponding to the angle of operation. In one aspect, the CCU 14 is configured to select the real image "RI" and a virtual image "VI" corresponding to the angle of operation of the imaging device 10, wherein the CCU 14 is further configured to process the real image "RI" and the virtual image "VI" to generate a three-dimensional image corresponding to the angle of operation. In another aspect, the CCU 14 is configured to select a pair of virtual images "VI1", "VI_{N}" corresponding to the angle of operation, wherein the CCU 14 is further configured to process the pair of selected virtual images "VI1", "VI_{N}" to generate a three-dimensional image corresponding to the angle of operation. It should be appreciated that the selected pair of virtual images "VI" need not be symmetrical to each other.

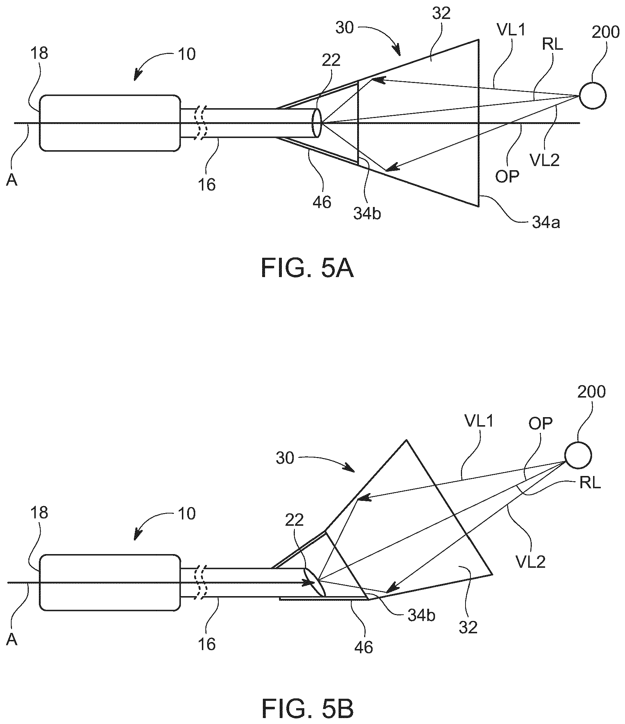

Referring now to the examples shown in FIGS. 5A-6C, various configurations of the imaging device 10 are provided. In each aspect, the reflective element 30 at least partially surrounds at the optical path. With reference first to FIGS. 5A-5C, the reflective element 30 may be coupled to and spaced apart from the distal end of the body using an attachment mechanism 46. The attachment mechanism 46 may be used to attach the reflective element 30 to the distal end of the scope portion 16 of the imaging device 10. The attachment mechanism 46 may be configured to maintain a set distance between the reflective element 30 and the optical system 20. In another aspect, the attachment mechanism 46 may be made of a resilient material to allow the reflective element 30 to be moved within the body of the patient. The attachment mechanism 46 may be formed of a memory alloy configured to retain a set configuration when physically manipulated but move to a different position when actuated. Thus, the orientation of the reflective element 30 may be moved with respect to the distal end of the scope portion 16.

FIGS. 5A and 5B depict an aspect where the attachment mechanism 46 permits movement of the reflective element 30 between a first position (see FIG. 3A) and a second position (see FIG. 3B). In the first position, the reflective element 30 is disposed circumferentially about and symmetrically with a longitudinal axis "A" of the scope portion 16. In the second position, the reflective element 30 is disposed at an angle relative to the longitudinal axis "A" of the scope portion 16, to facilitate a desired angle of operation. For example, in the example shown in FIG. 3B, the reflective element 30 is approximately thirty (30) degrees from longitudinal axis "A" of the scope portion 16 in the second position. However, it is contemplated that in the second position, the reflective element 30 may be disposed at any angle relative to the longitudinal axis "A" of the scope portion 16 to facilitate various angles of operation within the body of the patient. In another aspect, the attachment mechanism 46 is configured to retain the reflective element 30 at an angle with respect to the longitudinal axis "A" of the scope portion, and thus, the imaging device 10 is fixed in a position to look upwardly from the longitudinal axis "A" of the scope portion 16.

With reference again to FIG. 3A, an aspect of the reflective element 30 is shown where the reflective element 30 is a frustoconical member that is rigid and is centered along the longitudinal axis "A" of scope portion 16 of the imaging device 10 and the longitudinal axis "A" of the scope portion 16 is coaxial with the optical path "OP" of the imaging device 10. The attachment mechanism 46 is illustratively shown as being a frustoconical member having a first end which may be fitted onto the outer surface of the distal end of the scope portion 16 and a second end of the attachment mechanism 46 coupled to the reflective element 30. It should be appreciated that the second end of the attachment mechanism 46 can be coupled to the reflective element 30 using fastening techniques/mechanisms currently known or used in the art or later developed to include an adhesive, a thread and screw connection, a pin and slot arrangement, or the like. In such an aspect, the reflective element 30 is fixed in relationship with the endoscope.

It is contemplated that the reflective element 30 may be configured to be collapsible. Such an aspect may be advantageous to minimize the opening in which the endoscope may be introduced. In one aspect, the reflective element 30 is collapsed or stored within the distal end of the body and deployed by simply folding itself out of the distal end of the endoscope. In such an aspect, the reflective element 30 is formed of a shape memory alloy which is configured to fold from a stowed position to a deployed position. In another aspect, the reflective element 30 is a frustoconical member that includes a cut separating one end of the reflective element 30 from another. The reflective element 30 is formed of a shape memory alloy having a first memory position and a second memory position. In the first memory position, the reflective element 30 is formed into a roll, and in a second memory position, the reflective element 30 is formed into a conical shape. For example, the reflective element 30 may begin in the collapsed position prior to entering the body of the patient. Once inserted into the desired position by the medical personnel, the reflective element 30 may be actuated by application of an electric current or heat to move to the second memory position.

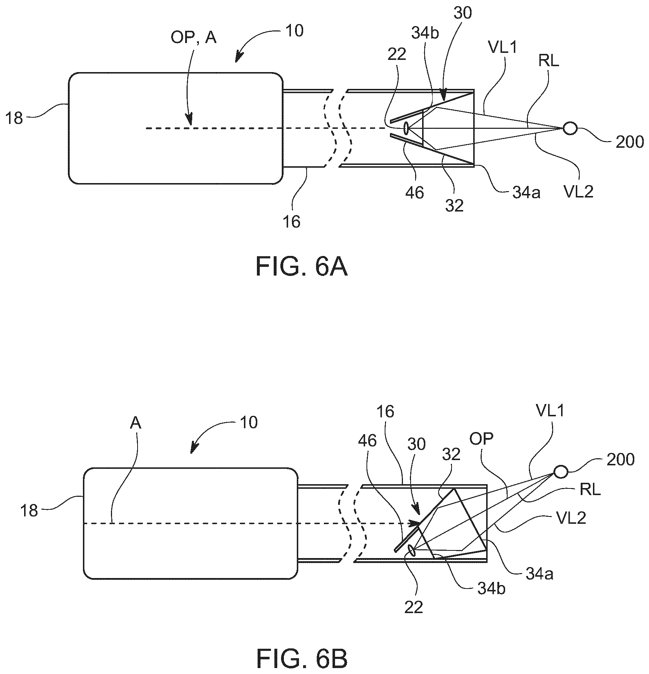

FIGS. 6A-6C, depict an aspect where the reflective element 30 is completely disposed within the distal end of the scope portion 16 of the imaging device 10 so as to reduce the footprint of the scope portion 16 relative to the aspects shown in FIGS. 5A-5C. The principles of the operation of the reflective element 30 and the CCU 14 remain the same as what was described with respect to FIGS. 5A-5C. FIG. 6A depicts an aspect where the reflective element 30 is centered within the scope portion 16 of the imaging device 10, and thus, the real light rays "RL" of the scene 200 are coaxial with the optical path "OP" producing a real image "RI" that is taken from the perspective of the imaging device 10. With the imaging device 10 shown in FIGS. 5A, virtual rays of light "VL1", "VL2" are reflected onto the image sensor 24 to generate a plurality of virtual images VI_{N}. FIG. 6B depicts an aspect where the reflective element 30 is angled, which may be preferable in instances where the endoscope is intended to provide a side view or a look up view. In such an aspect, the optical path "OP" is angled with respect to the longitudinal axis "A" of the scope portion 16 and is coaxial with the real light rays "RL", which is useful when the scene 200 is offset from the longitudinal axis of the scope portion 16.

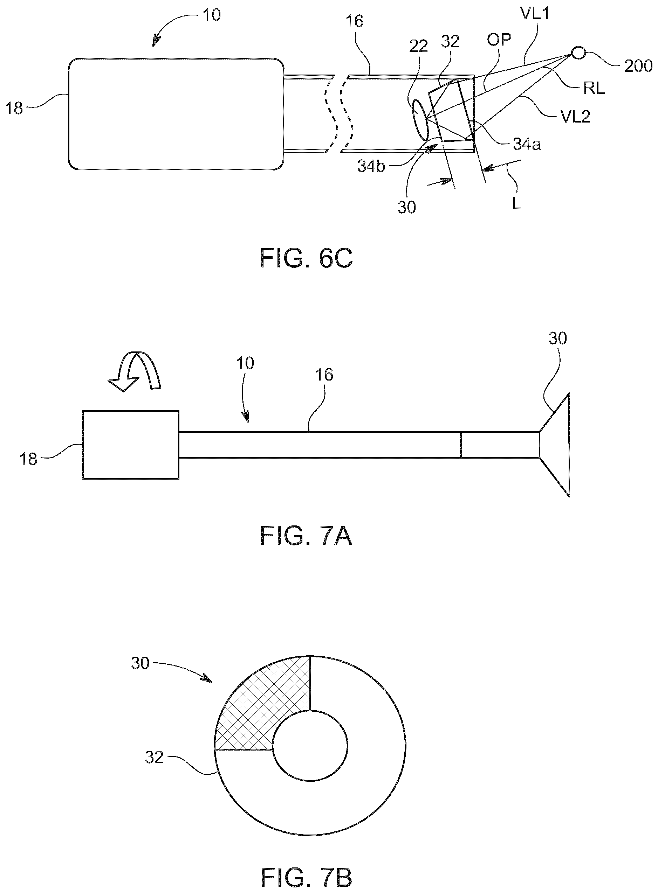

FIG. 6C depicts an aspect wherein the reflective element 30 has a length "L" smaller than the length of the reflective element 30 shown in FIGS. 5A and 5B, wherein the opening 34a disposed on a proximal end of the reflective element 30 is larger than its corresponding opening 34b shown in FIGS. 5A and 5B. In such an aspect, the objective lens assembly 22 includes a first lens that is larger than the first lens of the objective assembly shown in FIGS. 5A and 5B so as to collect all of the virtual light rays "VL1", "VL2" and "VL_{N}" reflected off of the reflective element 30, of which only "VL1" and "VL2" are pointed out. FIG. 6C illustrates that the dimensions disclosed herein are illustrative and not intended to limit the scope of the appended claims. To determine the optimal size of the reflective element 30, various parameters may be considered including the distance between the reflective element 30 and the objective lens assembly 22, the radius of the reflective element 30, the dimensions of the objective assembly, and the like.

The imaging device 10 may be configured to set a horizon. For instance, an image taken by the imaging device 10 held in the hand of a surgeon may be described as having a 0 degree rotation. As the image device is rotated, the image rotates. The CCU 14 may be further configured to process the angle of operation detected by the orientation detection unit 44 to select one of the virtual images "VI" having a perspective that corresponds to the angle of operation and process the selected virtual image "VI" with the real image "RI" to generate a three-dimensional image. The CCU 14 may be further configured to rotate the three-dimensional image so as to display the three-dimensional image in the set horizon.

Various known methods and computations may be processed by the CCU 14 to select one of the virtual images "VI" to process with the real image "RI" to generate a three-dimensional view. In one aspect, the CCU 14 may be configured to process a section of the distorted image "DI" to select a virtual image for generating the three-dimensional image. In such an aspect, the CCU 14 may be configured to use only a section of the reflective element 30 to support rotated perspectives. With reference now to FIGS. 7A and 7B, a description of such an aspect is provided. FIG. 7A shows the imaging device 10 being rotated clockwise 90 degrees. FIG. 7B depicts that the virtual light rays "VL_{N}" reflecting off of a quarter of the reflective element 30 (as indicated by the shading) are processed, wherein a virtual image generated by the reflection off of the shaded section is selected to generate a three-dimensional image. In particular, the section that is selected for image processing is determined by the degree of rotation and/or the desired perspective. For instance, the CCU 14 may be configured to interchange the virtual image VI1 with its symmetrical counterpart VI2, wherein virtual image VI1 represents a left-eye view and virtual image VI2 represents a right-eye view, and flipping/mirroring each view (180° rotation), resulting in mapping 0 degrees to 180 degrees perspectives, and 90 degrees to 270 degrees perspective, thus supporting a wider range of angles.

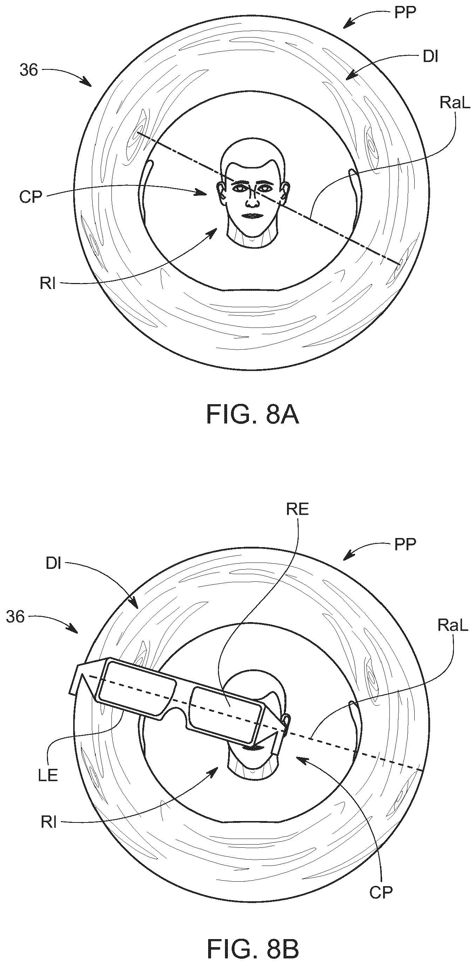

Referring now to the example shown in FIGS. 8A-8D, a description of the imaging device 10 for generating a three-dimensional image that is auto-leveled during a rotation of the imaging device 10 is provided. As discussed above, the reflective element 30 allows for capturing the scene 200 such that a real image "RI" is obtained simultaneously with a plurality of virtual images "VI" taken from a plurality of perspectives to form a composite image 36 within the real image. For example, in FIG. 8A, the top left image is an example of a composite image 36 showing the real image "RI" located in the central portion "CP" and the distorted image "DI" surrounding the real image "RI" located in the peripheral portion "PP". The distorted image "DI" is made of a plurality of virtual images "VI" of the scene 200 taken at different perspectives. In FIG. 8A, corresponding features of the real image "RI" and the virtual images "VI" appear along the radial line "RaL" which corresponds to a radial slice of the reflective element 30.

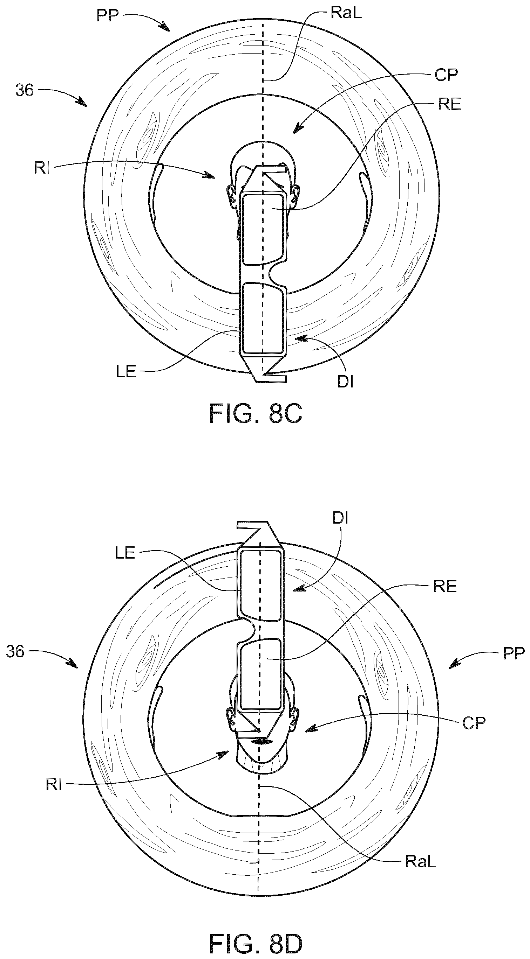

FIGS. 8B, 8C, and 8D depict the approximate position of left-eye "LE" and right-eye "RE" content (as indicated by the glasses) needed to create a stereoscopic synthesized views at different horizon rotations, as indicated by the three-dimensional glasses. In this aspect, the right eye "RE" view remains centered on the scene 200, illustrating the imaging device 10 being rotated counter-clockwise. The left eye "LE" view is centered on a virtual image of the composite image 36 along the radial line "RaL" which moves as the imaging device 10 is rotated. The CCU 14 may be configured to process the orientation or rotation of the imaging device 10 to determine the radial line "RaL" for which the CCU 14 may be further configured to select a virtual image found in the distorted image portion along the radial line "RaL" as indicated by the position of the left eye "LE" and process the selected virtual image "VI" to generated a three-dimensional image. The memory hardware 104 stores instructions correlating to known techniques and processes for determining which of the virtual images "VI" correspond to the angle of operation of the imaging device 10. Such techniques and process are disclosed in Non-Patent Literature entitled "Multiview Radial Catadioptric Imaging for Scene Capture" by Kuthirummal et al., and may include selecting a virtual image using a stereoscopic baseline (a physical separation distance between the left eye "LE" and right eye "RE" views) and taking a radial slice of the composite image at an angle corresponding to the rotation of the imaging device 10.

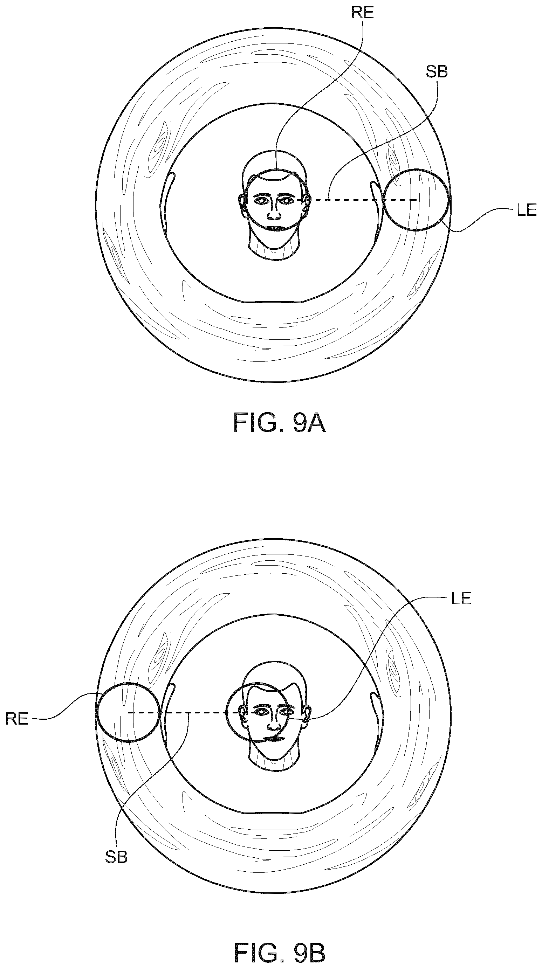

Additionally, the CCU 14 may be further configured to allow the video imaging system 100 to adjust the three-dimensional stereoscopic baseline "SB". For example, a synthesized three-dimensional image may be generated by processing the left eye "LE" view, which corresponds to the distorted image "DI", and the right eye "RE" view, which corresponds to the real image "RI" of the composite image 36, at different stereoscopic baselines "SB". For illustrative purposes, a description of adjusting the three-dimensional stereoscopic baseline "SB" is made with reference to the composite image 36 shown in FIG. 9A. In one aspect, the three-dimensional image may be generated by processing a pair of stereoscopic images. FIG. 9A illustrates one of the pair of stereoscopic images wherein the left eye "LE" image, which corresponds to a portion of the distorted image "DI", is taken at the three o'clock position, and the right eye "RE" image is of the real image "RI" at the center of the composite image 36.

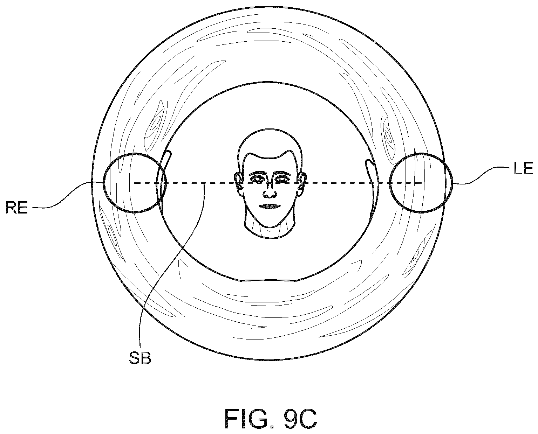

FIG. 9B illustrates the other of the pair of stereoscopic images wherein the left eye "LE" view is of the real image "RI" and the right eye "RE" image corresponds to a portion of the distorted image "DI" taken at the nine o'clock position. The stereoscopic base line "SB" of the two images are the same, but the view of the real image "RI" is taken at different positions, wherein the real image view of FIG. 9A is centered on the real image "RI" and the real image view of FIG. 9B is offset from the center of the real image "RI". Accordingly, the CCU 14 may be further configured to process the pair of images to obtain different perspectives of the real image "RI" and the virtual images "VI" wherein features from each of the images may be extracted and synthesized to generate a composite image 36 having various features such as texture enhancement. With reference now to FIG. 9C, the composite image 36 is shown being generated by a pair of virtual images "VI" disposed in the three o'clock and nine o'clock regions of the distorted image. The stereoscopic baseline "SB" shown in FIG. 9C is roughly twice the stereoscopic baseline "SB" shown in FIGS. 9A or 9B and results in a change in the overall depth scale of the composite image 36 relative to the composite images 36 generated in FIGS. 9A and 9B.

As stated above, the CCU 14 may be further configured to process the angle of operation of the imaging device 10 and select a pair of images corresponding to the angle of operation. The angle of operation of the imaging device 10 may be determined through information gathered from the orientation detection unit 44. However, it is also contemplated that the video image system 100 may include an input 48 for setting a desired perspective by the user. Any input 48, currently known or later developed may be adapted for use herein, illustratively including a button, keyboard, microphone, touch screen, or the like. Assume that the orientation detection unit 44 determines that the imaging device 10 has been rotated 30 degrees in a clockwise direction. The CCU 14 processes the 30 degree rotation to determine a radial line which corresponds to a radial slice of the composite image 36 taken at 30 degrees, in which case, the stereoscopic baseline "SB" of the imaging device 10 which corresponds to the radial slice is processed and a virtual image is taken along the stereoscopic baseline "SB" to select a virtual image "VI" or a real image "RI" (as the case may be) to generate a three-dimensional image that corresponds to the rotation.

In another aspect, the imaging device 10 of the CCU 14 is configured to process the desired perspective and the angle of operation to retain the image displayed in the desired perspective. For instance, the CCU 14 may be configured to retain the image displayed in a horizontal perspective. For illustrative purposes, assume that the horizontal perspective maintains an image in an upright position. Under such an assumption, the image is retained in an upright position as the imaging device 10 is turned. In such an aspect, the CCU 14 may be configured to process a difference in images between at least a pair of successive image frames 42 to determine the pair of images corresponding to the angle of operation. Additionally, the CCU 14 is configured to automatically perform stereoscopic image rotation by choosing a desired horizon and then determining the pair of images that correspond to the desired horizon. The desired horizon may correspond to the desired perspective of the user or to the angle of operation of the imaging device 10. Thus, as the imaging device 10 is rotated, the reflective element 30 is turned, in which case the real image "RI" is rotated as the imaging device 10 is rotated in the same degree in which the distorted image "DI" is rotated, assuming that the scene 200 is not rotated as well. The CCU 14 is configured to process the rotation to determine a radial slice of the composite image 36 that corresponds to the degree of rotation and the CCU 14 processes the distorted image "DI" to gather virtual images "VI" to generate a stereoscopic view in the desired horizon. For example, the CCU 14 processes the dimensions of the reflective element 30 and the parameters of the objective lens assembly 22 along with the actual rotation of the imaging device 10 to determine which of the virtual images "VI" are needed to generate an image that corresponds to the rotation of the real image "RI" using the principals described in "Multiview Radial Catadioptric Imaging for Scene Capture". In one aspect, the CCU 14 may select a pair of virtual images "VI" having the desired perspective. Alternatively, the CCU 14 may process the real image "RI" and rotate the real image "RI" to the desired perspective and process a virtual image "VI" having the desired perspective to generate a three-dimensional image in the desired perspective.

Further, the CCU 14 is configured to process the orientation of the imaging device 10 and select the left image and the right image from the plurality of images with the left image and the right image corresponding to the orientation of the imaging device 10. Additionally, processing the left image and the right image may include superimposing the left image onto the right image to create a stereoscopic image. More specifically, both the left image and the right image may include a first image and a second image, and the method may include the step of processing a difference in the first image and the second image to select a subsequent left view image and right view image. Accordingly, the CCU 14 is configured to automatically perform stereoscopic image rotation by processing the desired horizon and then determining the pair of images, real or virtual, that correspond to the desired horizon.

The CCU 14 may be further configured to detect an undesired illumination in the plurality of images, such as a reflection or glare, and select the pair of images that do not have a specular reflection, e.g. a glare or an increased illumination and correspond to the angle of operation, so as to reduce or remove glare by choosing a viewing angle where specular content is not reflected directly back into the optical system 20. For instance, in certain inter-body medical procedures, a light is used to illuminate the surgical scene in which case, a glare is generated by the reflection of the light off of wet anatomy, e.g. the specular reflection. As such, the CCU 14 is configured to process the data from the image sensor 24 to detect a specular reflection and selects the images that do not have said specular reflection. Further, if the direct view has a specular reflection blinding certain pixels, some of the view of the same scene 200 region from a different angle can avoid that glare. The image shown on the display 12 can be a composite of only the clearest views, e.g. virtual images "VI" that are free of or contains minimum glare, in such a case.

Additionally, the CCU 14 may be configured to extract dimensions of an object using triangulation. Triangulation is the process of determining the location of a point by forming triangles to the unknown point from known points. More specifically, the CCU 14 may analyze both the right image and the left image of the stereoscopic images using triangulation to extract dimensions of the scene 200, which may be compiled to generate a perspective view of the scene 200 different than the perspective of the imaging device 10.

In operation, the video imaging system 100 is configured to obtain a plurality of images from a plurality of perspectives from the reflective element 30. Additionally, the video imaging system 100 is configured to determine the orientation of the imaging device 10. For example, the orientation of the imaging device 10 may be gathered and/or determined using data from the inertial sensor. Next, the CCU 14 is configured to process the orientation of the imaging device 10 and select the left image and the right image from the plurality of images corresponding to the orientation of the imaging device 10. The left image and the right image may be a real image and or a virtual image that is disposed along a stereoscopic baseline "SB" which is taken along a radial slice of composite image 36 that corresponds to the detected orientation. In some examples, the left image and the right image include a first image and a second image, and the processing includes processing a difference in the first image and the second image to select a subsequent left view image and right view image. Further, in some examples, the processing includes superimposing the left image onto the right image to create a stereoscopic image. The stereoscopic image is then displayed on the display 12.

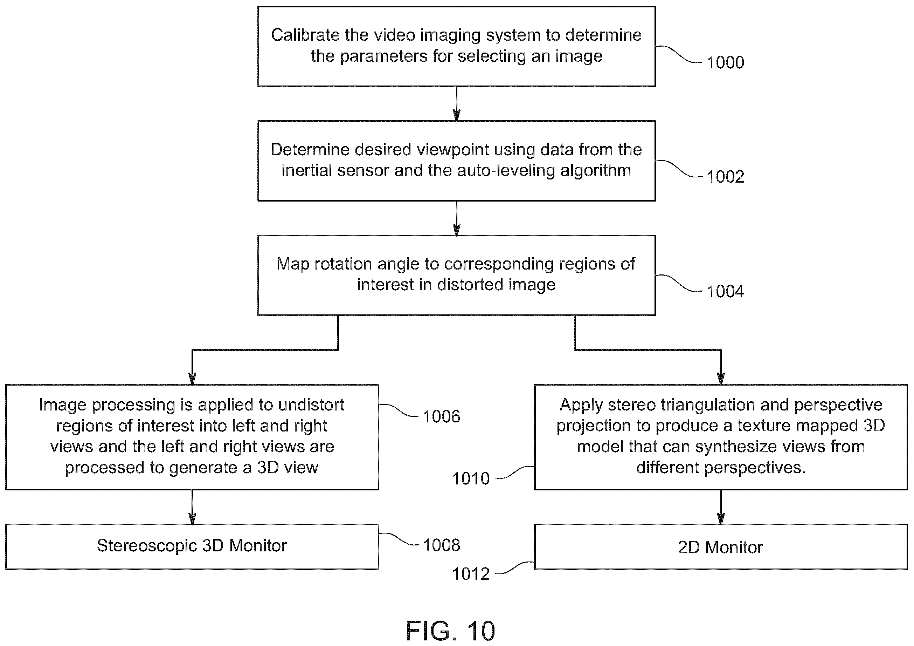

More specifically, as shown in FIG. 10, in operation, the objective lens assembly and the reflective element 30 are calibrated by the image system to determine the parameters to be used in determining the stereoscopic image selection at step 1000. Next, the image processor 14 determines the desired viewpoint using data from the inertial sensor and the auto-leveling algorithm at step 1002. Additionally, the rotation angle is mapped corresponding to regions of interest in the distorted image at step 1004. If the display 12 includes three-dimensional capabilities, the image processing is then applied to undistort regions of interest into left and right stereoscopic views at step 1006 and then displays the left and right stereoscopic views on the display 12 to form a three-dimensional image at the desired perspective at step 1008. However, if the display 12 includes only two-dimensional capabilities, the image processor 14 may apply stereo triangulation and perspective projection to produce a texture mapped mode that can synthesize views from various perspectives at step 1010 and displays the model on the display 12 at step 1012. For instance, the image processor, e.g. CCU 14 may be configured to select the virtual images "VI" that correspond to different perspectives of the scene 200 to generate different perspective views of the scene 200 using the principles described herein and set forth in "Multiview Radial Catadioptric Imaging for Scene Capture". The virtual images "VI" may be added onto the real image "RI" to generate a desired perspective. Alternatively, the desired perspective may be made wholly by the virtual images "VI". In such a manner a two-dimensional rendition of the scene may be generated in different perspectives. For instance, knowing a desired perspective, the virtual images "VI" that correspond to the desired perspective may be computed by the CCU 14 and compiled to generate the desired perspective view.

As an example, in an aspect where the imaging system is configured to generate a plurality of perspectives, the orientation of the imaging device 10 need not be processed. The imaging system 100 obtains images of an interior portion of a patient and the objective lens assembly 22 is configured to capture a real image along an optical axis defined by a longitudinal axis of the body. The reflective element 30 is configured to surround the optical axis. The reflective element 30 may be cylindrical or conical shaped so as to generate a plurality of virtual images from a plurality of perspectives which are captured by the image sensor 24. The CCU 14 processes the plurality of virtual images and the real image to generate a plurality of perspective view of the interior portion which are transmitted to the display 12. In such an aspect, an input such as a mouse or a touch screen may be used to change the perspective shown on the display 12. The CCU 14 processes the desired perspective transmitted by the input, and selects the virtual images "VI" and/or real image "RI" that correlates to the desired perspective. As such, a two-dimensional rendition of the interior portion of the patient may be viewed from different perspectives.

The video imaging system 100 as described herein obtains multiple perspective views of a scene 200 simultaneously using the optical lens 22 with the single image sensor 24 looking through the reflective element 30. The distorted image captured with the reflective element 30 includes the circular annulus of viewpoints from an offset perspective relative to the central view. By processing the distorted image, stereoscopic views can be obtained which are suitable for viewing on a three-dimensional monitor. Since the offset perspective views form a continuous circle around the central view, stereoscopic views corresponding to arbitrary horizon rotation angles can be extracted. This can be combined with an auto-leveling algorithm to support three-dimensional horizon stabilization by compensating for physical rotation of the imaging device 10 with synthetically rotated views providing clear viewing angles at every angle of operation.

The terminology used herein is for the purpose of describing particular exemplary configurations only and is not intended to be limiting. As used herein, the singular articles "a," "an," and "the" may be intended to include the plural forms as well, unless the context clearly indicates otherwise. The terms "comprises," "comprising," "including," and "having," are inclusive and therefore specify the presence of features, steps, operations, elements, and/or components, but do not preclude the presence or addition of one or more other features, steps, operations, elements, components, and/or groups thereof. The method steps, processes, and operations described herein are not to be construed as necessarily requiring their performance in the particular order discussed or illustrated, unless specifically identified as an order of performance. Additional or alternative steps may be employed.

A number of implementations have been described. Nevertheless, it will be understood that various modifications may be made without departing from the spirit and scope of the disclosure. Any such assembly is but a variation of the embodiments discussed above, and does not depart from the sprit and scope of the disclosure. Accordingly, other implementations are within the scope of the following claims.

## Claims

1. An imaging device (10) for obtaining an image of an interior portion of a patient, the imaging device (10) comprising:
a body (15) having a proximal end and a distal end configured to be inserted into the patient;
an optical system (20) including an image sensor (24) and an optical lens (22) configured to capture images along an optical axis (OA) defined by a longitudinal axis (AB) of the body (15); and
a reflective element (30) surrounding at least a portion of the optical axis (OA), wherein the reflective element (30) is cylindrically or conically shaped, and wherein the optical lens (22) is interposed between the image sensor (24) and the reflective element (30) and configured to transmit light from the reflective element (30) onto the image sensor (24).

2. The imaging device (10) of claim 1, wherein the reflective element (30) is attached to the distal end of the body (15).

3. The imaging device (10) of claim 1 or 2, wherein the reflective element (30) is spaced apart from the distal end.

4. The imaging device (10) of claim 1 or 2, wherein the reflective element (30) is at least partially disposed within the distal end.

5. The imaging device (10) of any of the preceding claims, wherein the reflective element (30) is configured to be moveable between a collapsed position and an extended position.

6. The imaging device (10) of any of the preceding claims, wherein the reflective element (30) is a cylindrical or conical mirror.

7. An imaging system (100) for obtaining images of an interior portion of a patient, the imaging system (100) comprising:
an imaging device (10) configured to operate at an angle of operation and including:
a body (15) having a proximal end and a distal end configured to be inserted into the interior portion of the patient;
an optical system (20) including an image sensor (24) and an optical lens (22) configured to capture images along an optical axis (OA) defined by a longitudinal axis (AB) of the body (15); and
a reflective element (30) configured to surround the optical axis, (OA) the reflective element (30) being one of a cylindrical or conical shape so as to generate a plurality of images from a plurality of perspectives, the plurality of images captured by the image sensor (24);
an image processor (14) configured to process the angle of operation of the imaging device (10) and select one of the plurality of images corresponding to the angle of operation, the selected image and a central image is processed to generate a three-dimensional image corresponding to the angle of operation; and
a display (12) configured to display the three-dimensional image.

8. The imaging system (100) of claim 7, further including an orientation detection unit (44) configured to sense the angle of operation of the imaging device (10).

9. The imaging system (100) of claim 7 or 8, wherein the image processor (14) is configured to select the pair of images according to a desired angle of operation of the imaging device (10).

10. The imaging system (100) of any of claims 7 to 9, further including an input (48) for setting a desired perspective, wherein the image processor (14) is configured to process the desired perspective and the angle of operation to select the pair of images.

11. The imaging system (100) of any of the preceding claims, wherein the image processor (14) is configured to process a difference in images between at least a pair of successive image frames to determine the pair of images corresponding to the angle of operation.

12. The imaging system (100) of any of the preceding claims, wherein the image processor (14) is configured to automatically perform stereoscopic image rotation by choosing a desired horizon and then determining the pair of images that correspond to the desired horizon.

13. The imaging system (100) of any of the preceding claims, wherein the image processor (14) is configured to extract dimensions of an object using triangulation.

14. The imaging system (100) of any of the preceding claims, wherein the image processor (14) is further configured to detect an illumination in a plurality of images and select the pair of images that do not have a specular reflection and correspond to the angle of operation.

15. A method of displaying an image from an imaging device (10) having a body (15), the method comprising the steps of:
obtaining a plurality of images from a plurality of perspectives from a reflective element (30), the reflective element (30) being one of a cylindrical or conical shape;
determining an orientation of the imaging device (10);
processing the orientation of the imaging device (10) and selecting a left image and a right image from the plurality of images, wherein one of the left image and the right image corresponds to a central image and the other of the left image and the right image is one of the plurality of images corresponding to the orientation of the imaging device (10); and
displaying the left image and the right image on a display.

16. The method of claim 15, wherein the step of processing the left image and the right image includes superimposing the left image onto the right image to create a three-dimensional image.

17. The method of claim 15 or 16, wherein both the left image and the right image includes a first image and a second image, and the method includes the step of processing a difference in the first image and the second image to select a subsequent left view image and right view image.

18. The method of any of the preceding claims, further comprising the step of analyzing the left image and the right image using triangulation to extract dimensions of an object.

19. The method of any of the preceding claims, further comprising the step of processing the plurality of images to select the left image and right image, wherein the selected left image and right image have an illumination that is not reflected directly into an optical lens (22).
